# EUROPEAN PATENT SPECIFICATION

(11) **EP 3 429 563 B1**
(45) Date of publication and mention of the grant of the patent: **26.04.2023**
(21) Application number: 17767319.1
(22) Date of filing: 14.03.2017
(51) Int. Cl.: A61K 31/4045, A61K 45/06, A61P 43/00, A61K 9/20, A61K 47/02

(54) **DESFORMYLFLUSTRABROMINE FOR USE IN THE TREATMENT OF OBSESSIVE-COMPULSIVE AND RELATED DISORDERS**
DESFORMYLFLUSTRABROMIN ZUR BEHANDLUNG VON ZWANGSSTÖRUNG UND VERWANDTEN ERKRANKUNGEN
DESFORMYLFLUSTRABROMINE DESTINÉ AU TRAITEMENT DU TROUBLE OBSESSIONAL COMPULSIF ET DES TROUBLES APPARENTÉS

(30) Priority: 15.03.2016 US 201662308560 P
(43) Date of publication of application: 23.01.2019
(73) Proprietor: Saint Joseph's University, Philadelphia, PA 19131 (US); University of Alaska Fairbanks, Fairbanks, AK 99775 (US); Virginia Commonwealth University, Richmond, VA 23219 (US)
(72) Inventor: SCHULTE, Marvin K., Philadelphia PA 19129 (US); GLENNON, Richard A., Midlothian VA 23113 (US); BULT-ITO, Abel, Fairbanks AK 99708 (US); KHATRI, Shailesh, Philadelphia PA 19104 (US); MITRA, Swarup, Fairbanks AK 99775 (US)
(74) Representative: Grünecker Patent- und Rechtsanwälte PartG mbB
(86) International application number: PCT/US2017/022271
(87) International publication number: WO 2017/160813

(56) References cited:
- WO-A1-2014/176460
- WO-A2-2004/034963
- US-A1- 2011 212 999
- SWARUP MITRA ET AL: "Attenuation of Compulsive-Like Behavior Through Positive Allosteric Modulation of [alpha]4[beta]2 Nicotinic Acetylcholine Receptors in Non-Induced Compulsive-Like Mice", FRONTIERS IN BEHAVIORAL NEUROSCIENCE, vol. 10, 5 January 2017 (2017-01-05), XP055624989, CH ISSN: 1662-5153, DOI: 10.3389/fnbeh.2016.00244
- SPRINGER ET AL.: 'Synthesis and activity of substituted carbamates as potentiators of the alpha- 4-beta-2 nicotinic acetylcholine receptor' BIOORGANIC & MEDICINAL CHEMISTRY LETTERS vol. 18, 2008, pages 5643 - 5647, XP025519206
- CAMPLING ET AL.: 'Acute Activation, Desensitization and Smoldering Activation of Human Acetylcholine Receptors' PLOS ONE. vol. 8, no. 11, 2013, page E79653, XP055423165
- Maegan M. Weltzin ET AL: "Pharmacological Characterization of the Allosteric Modulator Desformylflustrabromine and Its Interaction with [alpha]4[beta]2 Neuronal Nicotinic Acetylcholine Receptor Orthosteric Ligands", Journal of Pharmacology and Experimental Therapeutics, vol. 334, no. 3, 19 September 2010 (2010-09-19), pages 917-926, XP055722353, ISSN: 0022-3565, DOI: 10.1124/jpet.110.167684
- Swarup Mitra ET AL: "Attenuation of Compulsive-Like Behavior Through Positive Allosteric Modulation of ?4?2 Nicotinic Acetylcholine Receptors in Non-Induced Compulsive-Like Mice", Frontiers in Behavioral Neuroscience, vol. 10, 5 January 2017 (2017-01-05), XP055624989, CH ISSN: 1662-5153, DOI: 10.3389/fnbeh.2016.00244
- Anonymous: "Obsessive-Compulsive and Related Disorders" In: "Diagnostic and Statistical Manual of Mental Disorders, 5th. edition. (DSM-V)", 1 January 2013 (2013-01-01), American Psychiatric Association Publishing, XP055973234, pages 235-264, DOI: https://doi.org/10.1176/appi.books.9780890 425596,

## Description

### BACKGROUND OF THE INVENTION

Obsessive-compulsive disorder (OCD) is the fourth most common mental disorder, after addiction, depression, and social phobia. It has a lifetime prevalence of 1.6% in community surveys and 2.3% in the United States. OCD patients suffer from persistent obsessive thoughts (anxiety/distress) and compulsive repetitive behaviors to alleviate uncomfortable feelings of anxiety. OCD can have disabling effects throughout the patient's lifespan in both males and females.

Obsessions can be thematic, such as fear of contamination, pathological doubt, or need for symmetry/order, or somatic obsessions, like aggression. Repetitive compulsive behaviors involve washing, seeking, counting, sorting, hoarding and searching. Although OCD is classified as an anxiety disorder, many clinicians conceptualize the disease as a spectrum of related disorders (OCRD) sharing common clinical features of anxiety/fear and worry. OCRD encompasses a wide range of diseases, which includes somatoform (*e.g*., hypochondriasis), impulse control (*e.g*., trichotillomania, pathological gambling) and tic disorders (*e.g*., Tourette's syndrome). Repetitive behaviors in Autism Spectrum Disorders (ASD) have significant overlap with compulsive behaviors in OCD. Selective serotonin reuptake inhibitors (SSRIs), tricyclic antidepressants and cognitive behavioral therapy or their combination are often used as first line treatments. However, a large group of patients remain resistant to treatment either partially or completely.

Interestingly, pets and livestock also show problems with compulsive-like behaviors.

Compulsive-like acral lick in cats and dogs causes excessive licking that leads to large skin lesions, sores, and infections. Feather pecking in domestic hens and other poultry, parrots and other large birds in captivity leads to removal of large areas of feathers leading to deficiencies in thermoregulation and ability to fly. Feather pecking in laying hens is a major problem for the egg industry. Compulsive-like crib-biting and wood-chewing behaviors in horses are well documented. Crib-biting can lead to colic, *i.e*., abdominal pain, in horses, which can be a major problem for race horses. These types of compulsive-like behaviors are also being characterized in captive giraffe and okapi. There remains an unmet need in the art of novel compositions and methods of treating compulsive-like behaviors in pets and livestock.

US 2011/212999 discloses positive allosteric α4β2 acetylcholine receptor modulators for use in the treatment of a disease, disorder or condition which is responsive to the modulation of the cholinergic receptor, including obsessive compulsive disorders (OCD).

There remains an unmet need in the art for novel compositions and methods of treating obsessive compulsive disorder and compulsive-like behavior in a subject, such as but not limited to a human, pet or livestock. The present invention satisfies this unmet need.

### BRIEF SUMMARY OF THE INVENTION

The present invention provides desformylflustrabromine, a salt or solvate thereof, for use in a method of treating and/or preventing obsessive-compulsive and related disorders (OCRD) in a subject in need thereof.

In certain embodiments, the desformylflustrabromine is the only therapeutically effective compound administered to the subject. In other embodiments, the desformylflustrabromine is the only therapeutically effective compound administered in a therapeutically sufficient amount to treat and/or prevent the obsessive-compulsive and related disorders (OCRD).

In yet other embodiments, the subject had not previously received any other pharmacological treatment for the compulsive-like behavior and/or obsessive-compulsive related disorder or disease. In yet other embodiments, the subject had previously received another pharmacological treatment for the obsessive-compulsive and related disorders (OCRD). In yet other embodiments, the desformylflustrabromine is administered acutely to the subject. In yet other embodiments, the desformylflustrabromine is administered chronically to the

The effect of administering the desformylflustrabromine is to eliminate or reduce at least one symptom of the obsessive-compulsive and related disorders (OCRD) in the subject. The at least one symptom may be selected from the group consisting of anxiety, aggression, cognitive deficits, attention deficit, phobias, pathological doubt, lack of impulse control, tic disorders, substance abuse, somatic obsession and compulsive/pathological hoarding, washing, checking, counting, sorting, searching, eating, gambling, shopping, skin picking, hair picking, talking and sexual behavior.

In certain embodiments, the desformylflustrabromine is part of a pharmaceutical composition.

In certain embodiments, the pharmaceutical composition further comprises one or more additional agents known to treat symptoms of obsessive-compulsive and related disorders (OCRD).

In certain embodiments, the subject is further administered one or more additional agents known to treat symptoms of obsessive-compulsive and related disorders (OCRD). In other embodiments, the one or more additional agents are selected from the group consisting of clomipramine, fluvoxamine, fluoxetine, paroxetine, buspirone, sertraline, citalopram, escitalopram venlafaxine, dapoxetine, hydrocodone, D-cycloserine, buspirone, clonazepam, trazodone, tranylcypromine, venlafaxine, olanzapine, L-tryptophan, pindolol, gabapentin, lorazepam, bupropion, amantadine, methylphenidate, dexedrine, yohimbine, sildenafil, mirtazapine, nefazodone, valproate, lithium, risperidone, phenelzine, haloperidol, pimozide, aripiprazole, tramadol, N-acetylcysteine, topiramate, lamotrigine and inositol.

In certain embodiments, the desformylflustrabromine and the optional one or more additional agents are independently administered to the subject by at least one route selected from the group consisting of oral, nasal, inhalational, topical, buccal, rectal, pleural, peritoneal, vaginal, intramuscular, subcutaneous, transdermal, epidural, intratracheal, otic, intraocular, intrathecal, and intravenous routes.

In certain embodiments, the desformylflustrabromine is administered as part of a pharmaceutical composition. In other embodiments, the therapeutically effective amount of modulator ranges from 0.001 mg/day to 1,000 mg/day.

In certain embodiments, the subject is a mammal. In other embodiments, the subject is a bird. In yet other embodiments, the subject is a pet. In yet other embodiments, the subject is a livestock. In yet other embodiments, the subject is human.

In certain embodiments, the desformylflustrabromine is formulated for administration by at least one route selected from the group consisting of oral, nasal, inhalational, topical, buccal, rectal, pleural, peritoneal, vaginal, intramuscular, subcutaneous, transdermal, epidural, intratracheal, otic, intraocular, intrathecal, and intravenous routes.

### BRIEF DESCRIPTION OF THE DRAWINGS

The following detailed description of specific embodiments of the invention will be better understood when read in conjunction with the appended drawings. For the purpose of illustrating the invention, there are shown in the drawings specific embodiments. It should be understood, however, that the invention is not limited to the precise arrangements and instrumentalities of the embodiments shown in the drawings.

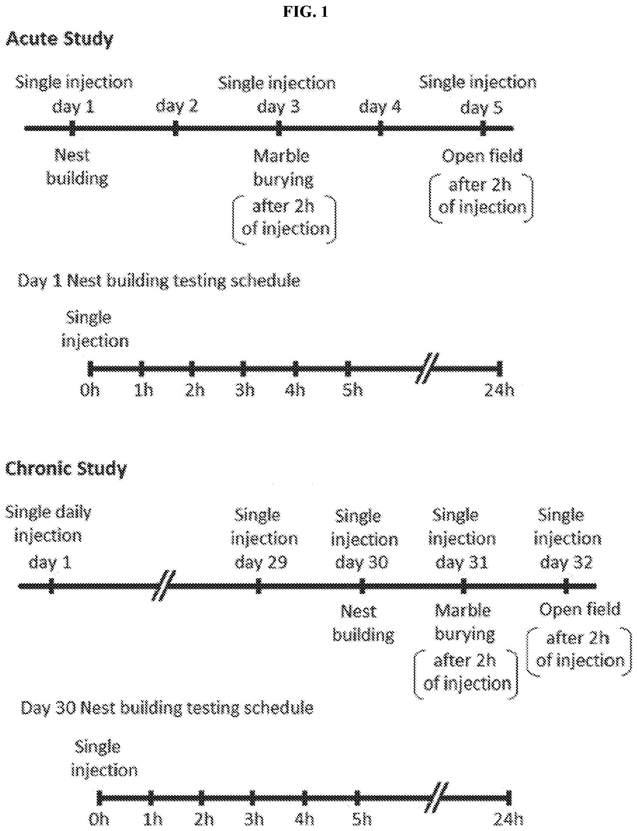

FIG. 1 is a schematic illustration of schedules for behavioral assessments following Desformylflustrabromine (dFBr) administration. Top Panel - Acute Study: mice in all experimental groups (0, 2, 4 and 6 mg/kg) received subcutaneous administration of vehicle or dFBr on days 1, 3 and 5. On day 1, immediately after injections all mice were subjected to nest-building and data were collected 1, 2, 3, 4, 5 and 24 h after injection (nest building testing schedule). On day 3 and 5, all mice were subjected to marble burying (MB) and open field (OF) behaviors, respectively, 2 h after vehicle or dFBr injections. On days 2 and 4, mice were not given injections and were not tested. Lower Panel - Chronic Study: for the chronic study mice from all groups (0, 2, 4 and 6 mg/kg) received daily single subcutaneous injections of vehicle or dFBr for 32 days. On day 30, immediately after injection all mice were subjected to nest-building and data were collected after 1, 2, 3, 4, 5 and 24 h after injection (nest building testing schedule). On day 31 and 32, all mice were subjected to MB and OF behaviors, respectively, 2 h after vehicle or dFBr injections

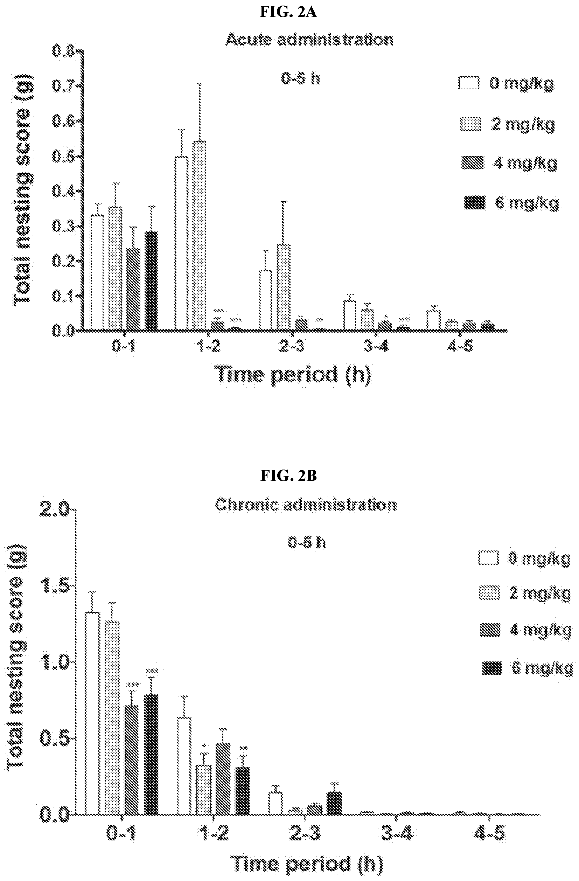

FIGs. 2A-2B illustrate the dose-dependent effect of dFBr on compulsive-like nesting behavior (NB) in compulsive-like male mice (n=12 in each group) from 1 to 5 hours of dFBr administration (FIG. 2A, acute administration; FIG. 2B, chronic administration). Data are expressed as the mean ± SEM for the amount of cotton used in grams. Statistical significance is considered as ^{∗}*p*<0.05, ^{∗∗}*p*<0.01 ^{∗∗∗}*p*<0.001. All comparisons are with respect to control (saline).

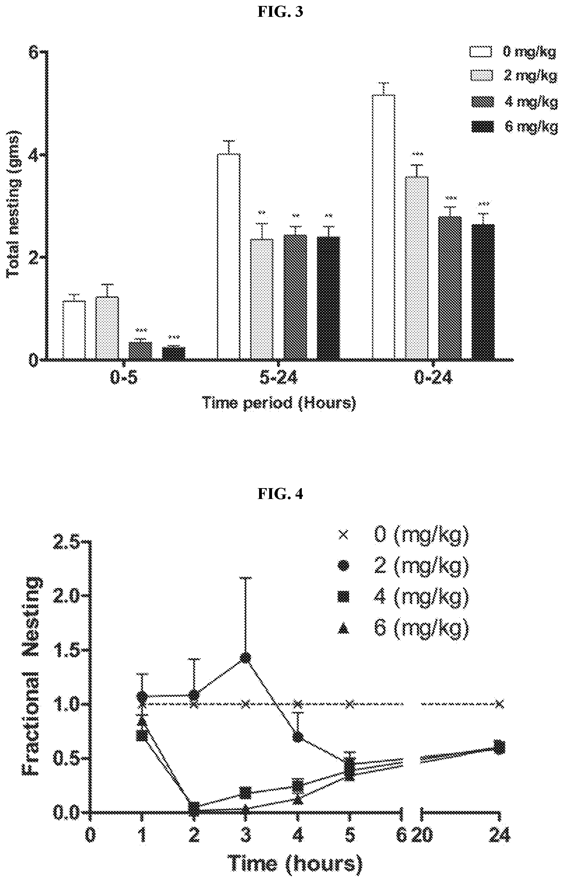

FIG. 3 illustrates the dose-dependent effects of dFBr on overall compulsive-like nesting behavior in compulsive-like male mice (n=12 in each group) between 0-5 hours, 5-24 hours, and 0-24 hours after dFBr administration. Data are expressed as the mean ± SEM for the amount of cotton used in grams. Statistical significance is considered as ^{∗∗}*p*<0.01 and ^{∗∗∗}*p*<0.001. All comparisons are with respect to control (saline).
FIG. 4 illustrates the overall dose dependent effect of dFBr on compulsive-like nesting behavior in compulsive-like male mice (n=12 in each group) from 0-24 hours of dFBr administration. Data are expressed as the mean ± SEM for the fractional nesting computed by dividing the average nesting for each dose group by the average of vehicle (saline).

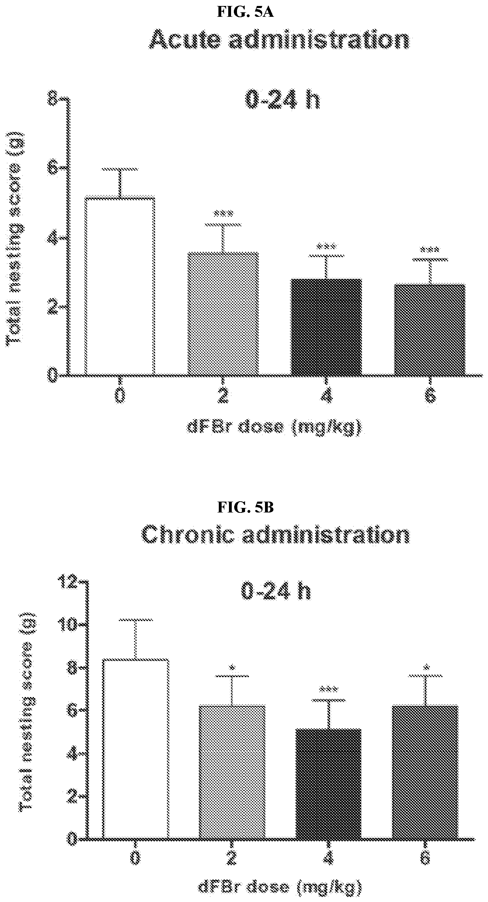

FIGs. 5A-5B illustrate dose-dependent effects of dFBr on overall compulsive-like NB behavior in compulsive-like BIG mice (n=12 in each group) 0-24 h after (FIG. 5A) acute and (FIG. 5B) chronic dFBr administration. Data are expressed as the mean ± SEM for the amount of cotton used in grams. Statistical significance is considered as ^{∗∗}*p*<0.05 and ^{∗∗∗}*p*<0.001. All comparisons are with respect to control (saline).

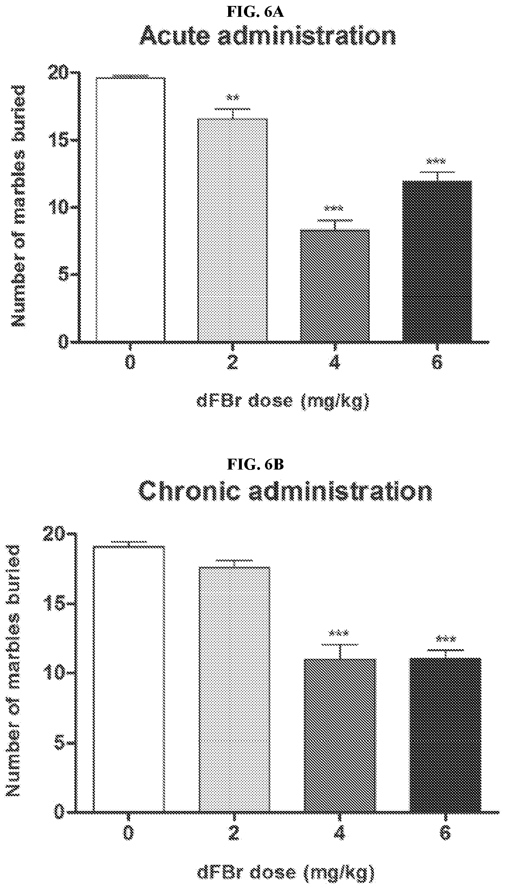

FIGs. 6A-6B illustrate the dose-dependent effect of dFBr on compulsive-like marble burying (MB) behavior in compulsive-like male mice (n=12 in each group) 2 hours after dFBr administration (FIG. 6A, acute administration; FIG. 6B, chronic administration). Data are expressed as the mean ± SEM for the number of marbles that are 2/3 buried. Statistical significance is considered as ^{∗∗}*p*<0.01 and ^{∗∗∗}*p*<0.001. All comparisons are with respect to control (saline).

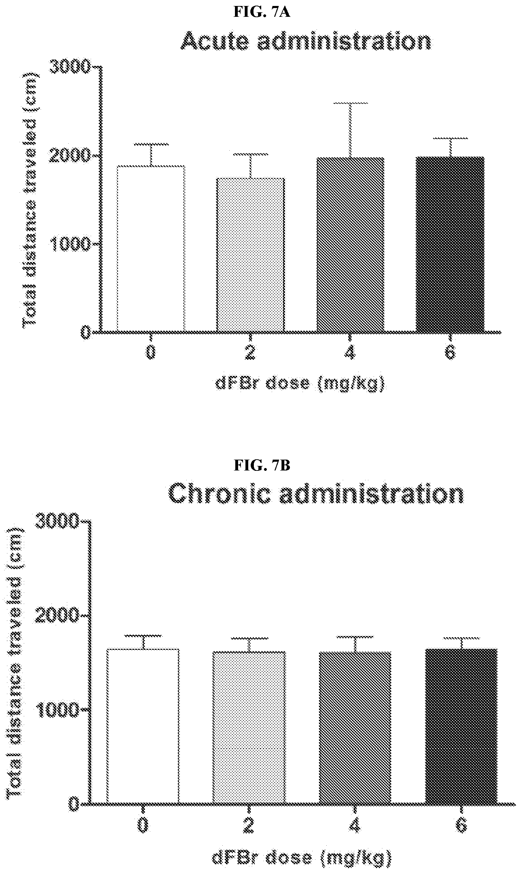

FIGs. 7A-7B illustrate the effect of dFBr on anxiety-like open field behavior in compulsive-like male mice (n=12 in each group) 2 hours after dFBr administration (FIG. 7A, acute administration; FIG. 7B, chronic administration). Data are expressed as the mean ± SEM for the total distance traveled in the open field. No statistical significance (*p*>0.05).

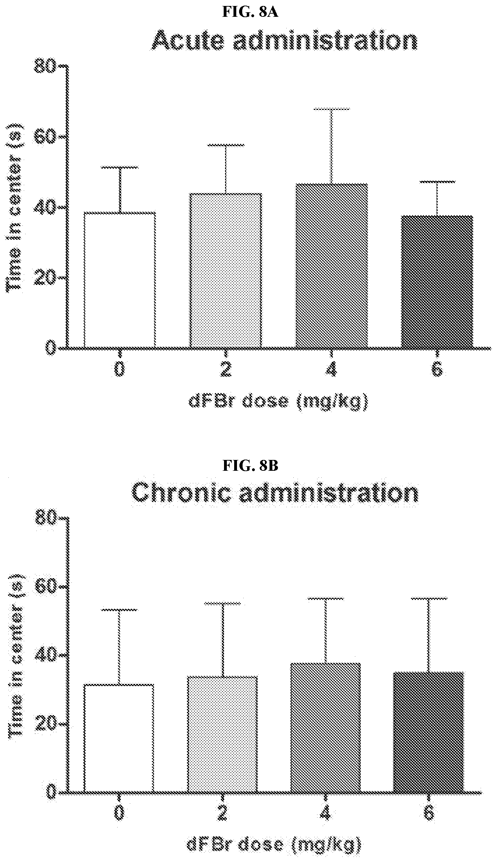

FIGs. 8A-8B illustrate the effect of dFBr on anxiety-like open field behavior in compulsive-like male mice (n=12 in each group) 2 hours after dFBr administration (FIG. 8A, acute administration; FIG. 8B, chronic administration). Data are expressed as the mean ± SEM for the time spent in the center of the open field. No statistical significance (*p*>0.05).

### DETAILED DESCRIPTION OF THE INVENTION

The present invention relates in part to the unexpected discovery that positive allosteric modulation of α4β2 nicotinic acetylcholine receptors leads to attenuation of compulsive-like behavior in a subject. Further, this modulation can be achieved by administration of desformylflustrabromine to the subject. In certain embodiments, the administration is acute. In other embodiments, the administration is chronic. In yet other embodiments, the administration comprises both acute and chronic phases.

The present invention discloses desformylflustrabromine, a salt or solvate thereof, and compositions for use in am method of treating and/or preventing obsessive-compulsive and related disorders (OCRD) in a subject.

The cholinergic system in the brain is comprised primarily of neuronal nicotinic acetylcholine receptors (nAChRs), members of the cys-loop superfamily of ligand gated ion channels. Alteration in expression profiles and modulation of nAChRs have been strongly associated with several neurological disorders. Strong evidence exists for cholinergic involvement in OCD, but there is controversial evidence about the effect of nicotinic activation on OCD symptoms. Glutamatergic hyperactivity associated with OCD may also be due to mediation of glutamate release by nicotinic receptor activation.

As demonstrated herein, allosteric α4β2 nicotinic acetylcholine receptor modulators can be used to treat OCD and other disorders that are part of the obsessive compulsive spectrum of disorders (OCRD). Allosteric α4β2 nicotinic receptor modulators collectively known as β2 receptor containing positive allosteric modulators or β2 PAMs, are small molecules that modulate the neuronal response of neuronal nicotinic receptors: they enhance agonist responses via increased agonist potency and/or efficacy. A typical β2 PAM produces an increase in the physiological effect of neural stimulation mediated via a specific subclass of receptors in the CNS that are responsive to nicotine and acetylcholine, namely those containing a β2 nicotinic receptor subunit (gene designation: *chrnb2*). The present invention discloses the use of β2 PAMs for the treatment of OCD, OCRD, and compulsive-like behaviors in ASD. The non-limiting prototypical β2 PAM used in this disclosure was desformylflustrabromine (dFBr). In a mouse model, subcutaneous injection of dFBr reduced OCD-like behaviors, thus validating the novel use of β2 PAMs in the treatment of the OCD spectrum of disorders.

Nest-building behavior in rodent models is homologous to hoarding in humans with OCD, which is considered to be a measure of compulsive-like behavior. The presently used model was achieved by bidirectionally selecting and breeding house mice, *Mus musculus,* for nest-building behavior. Bidirectional selection resulted in three lines of mice (with two replicate lines within each line) exhibiting three levels of nesting behavior. The two BIG lines consistently display high levels of nesting with a forty-fold difference in the amount of cotton used when compared to the two SMALL lines which display very low levels of nesting. The two randomly-bred lines serve as a selection control and show intermediate levels of nesting. The BIG lines of mice exhibit compulsive-like behavior (nest-building and marble burying) without gene manipulations, behavioral inductions, or administration of psychostimulants, which makes them a novel non-induced model for OCD. These mice are referred to as compulsive-like mice.

Positive allosteric modulators (PAMs) enhance agonist responses via increased agonist potency and/or efficacy. As demonstrated herein, PAMs of α4β2 nicotinic acetylcholine receptors (such as dFBr) attenuate compulsive-like behavior in valid rodent models. dFBr potentiates acetylcholine-induced whole cell responses by 370% for the high sensitivity (HS) and 260% for low sensitivity (LS) α4β2 receptors with an EC₅₀ of 40 µM and 2.5 µM respectively. As dFBr increases the efficacy of acetylcholine and does not directly activate receptors, its effect in the synapse may be to enhance acetylcholine mediated transmission. In the acute administration protocol, dFBr dose dependently attenuated NB and MB. Rapid effects (1-2 h after drug administration) of dFBr on MB and NB were observed for the chronic administration, which was in congruence with the acute study. Chronic administration also revealed sustained suppression of NB by dFBr following 5 weeks of treatment. In both the acute and chronic regimen, dFBr did not modulate OF behaviors.

Without wishing to be limited by any theory, application of dFBr, unlike application of exogenous agonists, can retain the control of synaptic activation via presynaptic release of acetylcholine, albeit with increased stimulation. The use of HS α4β2 receptors PAMs for the treatment of OCD has not been previously proposed or tested in any animal model. The present study demonstrates that dFBr, a novel PAM specific for α4β2 nAChRs and active at the HS α4β2 subtype, attenuates compulsive-like, but not general anxiety-like, behaviors in a non-induced compulsive-like mouse model. This demonstrates specificity of dFBr for the compulsive-like phenotype.

### Definitions

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, exemplary methods and materials are described. As used herein, each of the following terms has the meaning associated with it in this section.

Generally, the nomenclature used herein and the laboratory procedures in cell culture, molecular genetics, pharmacology and organic chemistry are those well-known and commonly employed in the art.

Standard techniques are used for biochemical and/or biological manipulations. The techniques and procedures are generally performed according to conventional methods in the art and various general references (*e.g*., Sambrook and Russell, 2012, Molecular Cloning, A Laboratory Approach, Cold Spring Harbor Press, Cold Spring Harbor, NY, and Ausubel et al., 2002, Current Protocols in Molecular Biology, John Wiley & Sons, NY), which are provided throughout this document.

The articles "a" and "an" are used herein to refer to one or to more than one *(i.e.,* to at least one) of the grammatical object of the article. By way of example, "an element" means one element or more than one element.

"About" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

A disease or disorder is "alleviated" if the severity or frequency of at least one sign or symptom of the disease or disorder experienced by a patient is reduced.

As used herein, the term "anxiety-like behavior" refers to an approach-avoidance conflict in an animal, such as a mammal, that is designed to inhibit an ongoing behavior that is characteristic for the animal, such as contrasting the tendency of the animal to engage in exploratory activity against the aversive properties of an open, brightly lit space.

As used herein, the term "compulsive-like behavior" refers to a repetitive behavior that is expressed in excess of what is normally expected in an animal, such as a mammal, including excessive repetitive front paw clawing motions and repetitive biting motions to pull cotton into a cage, and excessive digging behavior that buries marbles that were put on top of the bedding. Other excessive repetitive behaviors in an animal, such as a mammal or a bird, may include but are not limited to acral lick, pacing, crib biting, feather pulling, scratching, and hair pulling.

A "disease" is a state of health of an animal wherein the animal cannot maintain homeostasis, and wherein if the disease is not ameliorated then the animal's health continues to deteriorate. In contrast, a "disorder" in an animal is a state of health in which the animal is able to maintain homeostasis, but in which the animal's state of health is less favorable than it would be in the absence of the disorder. Left untreated, a disorder does not necessarily cause a further decrease in the animal's state of health.

The phrase "inhibit," as used herein, means to reduce a molecule, a reaction, an interaction, a gene, an mRNA, and/or a protein's expression, stability, function or activity by a measurable amount or to prevent entirely. Inhibitors are compounds that, *e.g*., bind to, partially or totally block stimulation, decrease, prevent, delay activation, inactivate, desensitize, or down regulate a protein, a gene, and an mRNA stability, expression, function and activity, *e.g*., antagonists.

As used herein, the term "modulate" means to exert a modifying or controlling influence.

As used herein, the term "pharmaceutically acceptable carrier" or "therapeutically acceptable carrier" means a pharmaceutically acceptable material, composition or carrier, such as a liquid or solid filler, stabilizer, dispersing agent, suspending agent, diluent, excipient, thickening agent, solvent or encapsulating material, involved in carrying or transporting a compound useful within the invention within or to the patient such that it may perform its intended function. Typically, such constructs are carried or transported from one organ, or portion of the body, to another organ, or portion of the body. Each carrier must be "acceptable" in the sense of being compatible with the other ingredients of the formulation, including the compound useful within the invention, and not injurious to the patient. Some examples of materials that may serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; surface active agents; alginic acid; pyrogen-free water; isotonic saline; Ringer's solution; ethyl alcohol; phosphate buffer solutions; and other non-toxic compatible substances employed in pharmaceutical formulations. As used herein, "pharmaceutically acceptable carrier" also includes any and all coatings, antibacterial and antifungal agents, and absorption delaying agents, and the like that are compatible with the activity of the compound useful within the invention, and are physiologically acceptable to the patient. Supplementary active compounds may also be incorporated into the compositions. The "pharmaceutically acceptable carrier" may further include a pharmaceutically acceptable salt of the compound useful within the invention. Other additional ingredients that may be included in the pharmaceutical compositions used in the practice of the invention are known in the art and described, for example in Remington's Pharmaceutical Sciences (Genaro, Ed., Mack Publishing Co., 1985, Easton, PA).

As used herein, the language "pharmaceutically acceptable salt" or "therapeutically acceptable salt" refers to a salt of the administered compounds prepared from pharmaceutically acceptable non-toxic acids, including inorganic acids, organic acids, solvates, hydrates, or clathrates thereof.

The terms "pharmaceutically effective amount" and "therapeutically effective amount" and "effective amount" refer to a nontoxic but sufficient amount of an agent to provide the desired biological result. That result can be reduction and/or alleviation of the signs, symptoms, or causes of a disease or disorder, or any other desired alteration of a biological system. An appropriate effective amount in any individual case may be determined by one of ordinary skill in the art using routine experimentation. By "pharmaceutical formulation" or "therapeutic formulation" it is further meant that the carrier, solvent, excipient(s) and/or salt must be compatible with the active ingredient of the formulation (*e.g*., a compound of the invention). It is understood by those of ordinary skill in this art that the terms "pharmaceutical formulation" and "pharmaceutical composition" are generally interchangeable, and they are so used for the purposes of this application.

As used herein, the term "prevent," "prevention," or "preventing" refers to any method to partially or completely prevent or delay the onset of one or more symptoms or features of a disease, disorder, and/or condition. Prevention is causing the clinical symptoms of the disease state not to develop, *i.e.,* inhibiting the onset of disease, in a subject that may be exposed to or predisposed to the disease state, but does not yet experience or display symptoms of the disease state. Prevention may be administered to a subject who does not exhibit signs of a disease, disorder, and/or condition.

As used herein, the term "subject," "patient" or "individual" to which administration is contemplated includes, but is not limited to, humans (*i.e.,* a male or female of any age group, *e.g.,* a pediatric subject (*e.g.,* infant, child, adolescent) or adult subject (*e.g*., young adult, middle-aged adult or senior adult)) and/or other primates (*e.g*., cynomolgus monkeys, rhesus monkeys); mammals, including commercially relevant mammals such as cattle, pigs, horses, sheep, goats, cats, and/or dogs; and/or birds, including commercially relevant birds such as chickens, ducks, geese, quail, and/or turkeys. The term "subject" also refers to pets and livestock, including, but not being limited to, the animals listed elsewhere herein.

The terms "treat," "treating," and "treatment," refer to therapeutic or preventative measures described herein. The methods of "treatment" employ administration to a subject, in need of such treatment, a composition of the present invention, for example, a subject afflicted a disease or disorder, or a subject who ultimately may acquire such a disease or disorder, in order to prevent, cure, delay, reduce the severity of, or ameliorate one or more symptoms of the disorder or recurring disorder, or in order to prolong the survival of a subject beyond that expected in the absence of such treatment.

The following abbreviations are used herein: ADHD, attention deficit hyperactivity disorder; ASD, autism spectrum disorders; dFBr, desformylflustrabromine, or a salt or solvate thereof; fMRI, functional magnetic resonance imaging; HS, high acetylcholine sensitivity; LS, low acetylcholine sensitivity; MB, marble burying; nAChRs, nicotinic acetylcholine receptors; NB, nest building; OCD, obsessive-compulsive disorder; OCRD, obsessive-compulsive related disorder; OF, open field; PAM, positive allosteric modulator; PET, positron emission tomography; SSRIs, selective serotonin reuptake inhibitors.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed sub ranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

### Compounds and Compositions

The positive allosteric α4β2 nicotinic acetylcholine receptor modulator for use in a method of treating and/or preventing obsessive/compulsive and related disorders (OCRD) is desformylflustrabromine (also known as 2-[6-bromo-2-(2-methylbut-3-en-2-yl)-1H-indol-3-yl]-N-methylethanamine or dFBr):

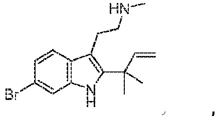

In certain embodiments, the compounds of the invention exist as tautomers. All tautomers are included within the scope of the compounds recited herein.

The present invention includes pharmaceutical compositions comprising desformylflustrabromine. The pharmaceutical compositions described herein may be prepared by any method known or hereafter developed in the art of pharmacology. In general, such preparatory methods include bringing the active ingredient into association with a carrier or one or more other accessory ingredients, and then, if necessary or desirable, shaping or packaging the product into a desired single- or multidose unit.

### Salts

The compounds described herein may form salts with acids and/or bases, and such salts are included in the present invention. In certain embodiments, the salts are pharmaceutically acceptable salts. The term "salts" embraces addition salts of free acids and/or basis that are useful within the methods of the invention. The term "pharmaceutically acceptable salt" refers to salts that possess toxicity profiles within a range that affords utility in pharmaceutical applications. Pharmaceutically unacceptable salts may nonetheless possess properties such as high crystallinity, which have utility in the practice of the present invention, such as for example utility in process of synthesis, purification or formulation of compounds useful within the methods of the invention.

Suitable pharmaceutically acceptable acid addition salts may be prepared from an inorganic acid or from an organic acid. Examples of inorganic acids include hydrochloric, hydrobromic, hydriodic, nitric, carbonic, sulfuric (including sulfate and hydrogen sulfate), and phosphoric acids (including hydrogen phosphate and dihydrogen phosphate). Appropriate organic acids may be selected from aliphatic, cycloaliphatic, aromatic, araliphatic, heterocyclic, carboxylic and sulfonic classes of organic acids, examples of which include formic, acetic, propionic, succinic, glycolic, gluconic, lactic, malic, tartaric, citric, ascorbic, glucuronic, maleic, malonic, saccharin, fumaric, pyruvic, aspartic, glutamic, benzoic, anthranilic, 4-hydroxybenzoic, phenylacetic, mandelic, embonic (pamoic), methanesulfonic, ethanesulfonic, benzenesulfonic, pantothenic, trifluoromethanesulfonic, 2-hydroxyethanesulfonic, p-toluenesulfonic, sulfanilic, cyclohexylaminosulfonic, stearic, alginic, β-hydroxybutyric, salicylic, galactaric and galacturonic acid.

Suitable pharmaceutically acceptable base addition salts of compounds of the invention include, for example, metallic salts including alkali metal, alkaline earth metal and transition metal salts such as, for example, calcium, magnesium, potassium, sodium and zinc salts. Pharmaceutically acceptable base addition salts also include organic salts made from basic amines such as, for example, N,N'-dibenzylethylene-diamine, chloroprocaine, choline, diethanolamine, ethylenediamine, meglumine (also known as N-methylglucamine) and procaine. All of these salts may be prepared from the corresponding compound by reacting, for example, the appropriate acid or base with the compound.

### Combination Therapies

In certain embodiments, the compounds of the invention are useful in the methods of present invention when used concurrently with at least one additional compound useful for treating compulsive-like behavior, obsessive-compulsive related disorders and diseases, or compulsive-like behaviors in ASD. In other embodiments, the compounds of the invention are useful in the methods of the present invention in combination with at least one additional compound useful for treating compulsive-like behavior.

These additional compounds may comprise compounds of the present invention or other compounds, such as commercially available compounds, known to treat compulsive-like behavior or symptoms. In certain embodiments, the combination of at least one compound of the invention, or a salt, solvate, enantiomer, diastereoisomer or tautomer thereof, and at least one additional compound useful for treating compulsive-like behavior or symptoms has additive, complementary or synergistic effects in the treatment of compulsive-like behavior or symptoms.

In a non-limiting example, the compounds of the invention may be used concurrently or in combination with antidepressants. In a non-limiting example, the compounds of the invention may be used concurrently or in combination with one or more of the following drugs: clomipramine, fluvoxamine, fluoxetine, paroxetine, buspirone, sertraline, citalopram, escitalopram venlafaxine, dapoxetine, hydrocodone, D-cycloserine, buspirone, clonazepam, trazodone, tranylcypromine, venlafaxine, olanzapine, L-tryptophan, pindolol, gabapentin, lorazepam, bupropion, amantadine, methylphenidate, dexedrine, yohimbine, sildenafil, mirtazapine, nefazodone, valproate, lithium, risperidone, phenelzine, haloperidol, pimozide, aripiprazole, tramadol, N-acetylcysteine, topiramate, lamotrigine and inositol.

A synergistic effect may be calculated, for example, using suitable methods such as, for example, the Sigmoid-Eₘₐₓ equation (Holford & Scheiner, 1981, Clin. Pharmacokinet. 6:429-453), the equation of Loewe additivity (Loewe & Muischnek, 1926, Arch. Exp. Pathol Pharmacol. 114: 313-326) and the median-effect equation (Chou & Talalay, 1984, Adv. Enzyme Regul. 22:27-55). Each equation referred to elsewhere herein may be applied to experimental data to generate a corresponding graph to aid in assessing the effects of the drug combination. The corresponding graphs associated with the equations referred to elsewhere herein are the concentration-effect curve, isobologram curve and combination index curve, respectively.

In certain embodiments, the desformylflustrabromine is administered to the subject by at least one route selected from the group consisting of oral, nasal, inhalational, topical, buccal, rectal, pleural, peritoneal, vaginal, intramuscular, subcutaneous, transdermal, epidural, intratracheal, otic, intraocular, intrathecal, and intravenous routes. In other embodiments, the modulator is administered as part of a pharmaceutical composition.

In certain embodiments, the therapeutically effective amount of desformylflustrabromine ranges from 0.001 mg/day to 1,000 mg/day. In certain embodiments, the therapeutically effective frequency of dosing of modulator ranges from intermittent, about 1 to about 5 times a day or more, to continuous administration.

In certain embodiments, the subject is a mammal or a bird. In other embodiments, the subject is a pet, livestock or human.

### Administration/Dosage/Formulations

The regimen of administration may affect what constitutes an effective amount. The therapeutic formulations may be administered to the subject either prior to or after the onset of a disease or disorder contemplated in the invention. Further, several divided dosages, as well as staggered dosages may be administered daily or sequentially, or the dose may be continuously infused, or may be a bolus injection. Further, the dosages of the therapeutic formulations may be proportionally increased or decreased as indicated by the exigencies of the therapeutic or prophylactic situation.

Administration of the compositions of the present invention to a patient, preferably a mammal, more preferably a human, may be carried out using known procedures, at dosages and for periods of time effective to treat a disease or disorder contemplated in the invention. An effective amount of the therapeutic compound necessary to achieve a therapeutic effect may vary according to factors such as the state of the disease or disorder in the patient; the age, sex, and weight of the patient; and the ability of the therapeutic compound to treat a disease or disorder contemplated in the invention. Dosage regimens may be adjusted to provide the optimum therapeutic response. For example, several divided doses may be administered daily or the dose may be proportionally reduced as indicated by the exigencies of the therapeutic situation. A non-limiting example of an effective dose range for a therapeutic compound of the invention is from 1 and 5,000 mg/kg of body weight/per day. The pharmaceutical compositions useful for practicing the invention may be administered to deliver a dose of from 1 ng/kg/day and 100 mg/kg/day. One of ordinary skill in the art would be able to study the relevant factors and make the determination regarding the effective amount of the therapeutic compound without undue experimentation.

Actual dosage levels of the active ingredients in the pharmaceutical compositions of this invention may be varied so as to obtain an amount of the active ingredient that is effective to achieve the desired therapeutic response for a particular patient, composition, and mode of administration, without being toxic to the patient.

In particular, the selected dosage level depends upon a variety of factors including the activity of the particular compound employed, the time of administration, the rate of excretion of the compound, the duration of the treatment, other drugs, compounds or materials used in combination with the compound, the age, sex, weight, condition, general health and prior medical history of the patient being treated, and like factors well known in the medical arts.

A medical doctor, e.g., physician or veterinarian, having ordinary skill in the art may readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved.

In particular embodiments, it is advantageous to formulate the compound in dosage unit form for ease of administration and uniformity of dosage. Dosage unit form as used herein refers to physically discrete units suited as unitary dosages for the patients to be treated; each unit containing a predetermined quantity of therapeutic compound calculated to produce the desired therapeutic effect in association with the required pharmaceutical vehicle. The dosage unit forms of the invention are dictated by and directly dependent on (a) the unique characteristics of the therapeutic compound and the particular therapeutic effect to be achieved, and (b) the limitations inherent in the art of compounding/formulating such a therapeutic compound for the treatment of a disease or disorder contemplated in the invention.

In certain embodiments, the compositions of the invention are formulated using one or more pharmaceutically acceptable excipients or carriers. In other embodiments, the pharmaceutical compositions of the invention comprise a therapeutically effective amount of a compound of the invention and a pharmaceutically acceptable carrier. In yet other embodiments, the compound of the invention is the only biologically active agent (*i.e.,* capable of treating compulsive-like behavior, treating obsessive compulsive disorder symptoms) in the composition. In yet other embodiments, the compound of the invention is the only biologically or therapeutically active agent (*i.e.,* capable of treating compulsive-like behavior, treating obsessive compulsive disorder symptoms) in therapeutically effective amounts in the composition.

The carrier may be a solvent or dispersion medium containing, for example, water, ethanol, polyol (for example, glycerol, propylene glycol, and liquid polyethylene glycol, and the like), suitable mixtures thereof, and vegetable oils. The proper fluidity may be maintained, for example, by the use of a coating such as lecithin, by the maintenance of the required particle size in the case of dispersion and by the use of surfactants. Prevention of the action of microorganisms may be achieved by various antibacterial and antifungal agents, for example, parabens, chlorobutanol, phenol, ascorbic acid, thimerosal, and the like. In many cases, it is preferable to include isotonic agents, for example, sugars, sodium chloride, or polyalcohols such as mannitol and sorbitol, in the composition. Prolonged absorption of the injectable compositions may be brought about by including in the composition an agent that delays absorption, for example, aluminum monostearate or gelatin.

In certain embodiments, the compositions of the invention are administered to the patient in dosages that range from one to five times per day or more. In other embodiments, the compositions of the invention are administered to the patient in range of dosages that include, but are not limited to, once every day, every two days, every three days to once a week, and once every two weeks. It is readily apparent to one skilled in the art that the frequency of administration of the various combination compositions of the invention varies from individual to individual depending on many factors including, but not limited to, age, disease or disorder to be treated, gender, overall health, and other factors. Thus, the invention should not be construed to be limited to any particular dosage regime and the precise dosage and composition to be administered to any patient is determined by the attending physical taking all other factors about the patient into account.

Desformylflustrabromine for administration may be in the range of from about 1 µg to about 10,000 mg, about 20 µg to about 9,500 mg, about 40 µg to about 9,000 mg, about 75 µg to about 8,500 mg, about 150 µg to about 7,500 mg, about 200 µg to about 7,000 mg, about 3050 µg to about 6,000 mg, about 500 µg to about 5,000 mg, about 750 µg to about 4,000 mg, about 1 mg to about 3,000 mg, about 10 mg to about 2,500 mg, about 20 mg to about 2,000 mg, about 25 mg to about 1,500 mg, about 30 mg to about 1,000 mg, about 40 mg to about 900 mg, about 50 mg to about 800 mg, about 60 mg to about 750 mg, about 70 mg to about 600 mg, about 80 mg to about 500 mg, and any and all whole or partial increments therebetween.

In some embodiments, the dose of a compound of the invention is from about 1 mg and about 2,500 mg. In some embodiments, a dose of a compound of the invention used in compositions described herein is less than about 10,000 mg, or less than about 8,000 mg, or less than about 6,000 mg, or less than about 5,000 mg, or less than about 3,000 mg, or less than about 2,000 mg, or less than about 1,000 mg, or less than about 500 mg, or less than about 200 mg, or less than about 50 mg. Similarly, in some embodiments, a dose of a second compound as described herein is less than about 1,000 mg, or less than about 800 mg, or less than about 600 mg, or less than about 500 mg, or less than about 400 mg, or less than about 300 mg, or less than about 200 mg, or less than about 100 mg, or less than about 50 mg, or less than about 40 mg, or less than about 30 mg, or less than about 25 mg, or less than about 20 mg, or less than about 15 mg, or less than about 10 mg, or less than about 5 mg, or less than about 2 mg, or less than about 1 mg, or less than about 0.5 mg, and any and all whole or partial increments thereof.

In certain embodiments, the present invention is directed to a packaged pharmaceutical composition comprising a container holding a therapeutically effective amount of a compound of the invention, alone or in combination with a second pharmaceutical agent; and instructions for using the compound to treat, prevent, or reduce one or more symptoms of a disease or disorder contemplated in the invention.

Formulations may be employed in admixtures with conventional excipients, *i.e.,* pharmaceutically acceptable organic or inorganic carrier substances suitable for oral, parenteral, nasal, intravenous, subcutaneous, enteral, or any other suitable mode of administration, known to the art. The pharmaceutical preparations may be sterilized and if desired mixed with auxiliary agents, *e.g.,* lubricants, preservatives, stabilizers, wetting agents, emulsifiers, salts for influencing osmotic pressure buffers, coloring, flavoring and/or aromatic substances and the like. They may also be combined where desired with other active agents.

Routes of administration of any of the compositions of the invention include oral, nasal, rectal, intravaginal, parenteral, buccal, sublingual or topical. The compounds for use in the invention may be formulated for administration by any suitable route, such as for oral or parenteral, for example, transdermal, transmucosal (*e.g.,* sublingual, lingual, (trans)buccal, (trans)urethral, vaginal (*e.g.,* trans- and perivaginally), (intra)nasal and (trans)rectal), intravesical, intrapulmonary, intraduodenal, intragastrical, intrathecal, subcutaneous, intramuscular, intradermal, intra-arterial, intravenous, intrabronchial, inhalation, and topical administration.

Suitable compositions and dosage forms include, for example, tablets, capsules, caplets, pills, gel caps, troches, dispersions, suspensions, solutions, syrups, granules, beads, transdermal patches, gels, powders, pellets, magmas, lozenges, creams, pastes, plasters, lotions, discs, suppositories, liquid sprays for nasal or oral administration, dry powder or aerosolized formulations for inhalation, compositions and formulations for intravesical administration and the like. It should be understood that the formulations and compositions that would be useful in the present invention are not limited to the particular formulations and compositions that are described herein.

### Oral Administration

For oral application, particularly suitable are tablets, dragees, liquids, drops, suppositories, or capsules, caplets and gelcaps. The compositions intended for oral use may be prepared according to any method known in the art and such compositions may contain one or more agents selected from the group consisting of inert, non-toxic pharmaceutically excipients that are suitable for the manufacture of tablets. Such excipients include, for example an inert diluent such as lactose; granulating and disintegrating agents such as cornstarch; binding agents such as starch; and lubricating agents such as magnesium stearate. The tablets may be uncoated or they may be coated by known techniques for elegance or to delay the release of the active ingredients. Formulations for oral use may also be presented as hard gelatin capsules wherein the active ingredient is mixed with an inert diluent.

For oral administration, the compounds of the invention may be in the form of tablets or capsules prepared by conventional means with pharmaceutically acceptable excipients such as binding agents (*e.g.,* polyvinylpyrrolidone, hydroxypropylcellulose or hydroxypropylmethylcellulose); fillers (*e.g.,* cornstarch, lactose, microcrystalline cellulose or calcium phosphate); lubricants (*e.g.,* magnesium stearate, talc, or silica); disintegrates (*e.g.,* sodium starch glycollate); or wetting agents (*e.g.,* sodium lauryl sulphate). If desired, the tablets may be coated using suitable methods and coating materials such as OPADRY^{™} film coating systems available from Colorcon, West Point, Pa. (*e.g.,* OPADRY^{™} OY Type, OYC Type, Organic Enteric OY-P Type, Aqueous Enteric OY-A Type, OY-PM Type and OPADRY^{™} White, 32K18400). Liquid preparation for oral administration may be in the form of solutions, syrups or suspensions. The liquid preparations may be prepared by conventional means with pharmaceutically acceptable additives such as suspending agents (*e.g.,* sorbitol syrup, methyl cellulose or hydrogenated edible fats); emulsifying agent (*e.g.,* lecithin or acacia); non-aqueous vehicles (*e.g.,* almond oil, oily esters or ethyl alcohol); and preservatives (*e.g.,* methyl or propyl p-hydroxy benzoates or sorbic acid).

Granulating techniques are well known in the pharmaceutical art for modifying starting powders or other particulate materials of an active ingredient. The powders are typically mixed with a binder material into larger permanent free-flowing agglomerates or granules referred to as a "granulation". For example, solvent-using "wet" granulation processes are generally characterized in that the powders are combined with a binder material and moistened with water or an organic solvent under conditions resulting in the formation of a wet granulated mass from which the solvent must then be evaporated.

Melt granulation generally consists in the use of materials that are solid or semi-solid at room temperature (*i.e.,* having a relatively low softening or melting point range) to promote granulation of powdered or other materials, essentially in the absence of added water or other liquid solvents. The low melting solids, when heated to a temperature in the melting point range, liquefy to act as a binder or granulating medium. The liquefied solid spreads itself over the surface of powdered materials with which it is contacted, and on cooling, forms a solid granulated mass in which the initial materials are bound together. The resulting melt granulation may then be provided to a tablet press or be encapsulated for preparing the oral dosage form. Melt granulation improves the dissolution rate and bioavailability of an active (*i.e*., drug) by forming a solid dispersion or solid solution.

U.S. Patent No. 5,169,645 discloses directly compressible wax-containing granules having improved flow properties. The granules are obtained when waxes are admixed in the melt with certain flow improving additives, followed by cooling and granulation of the admixture. In certain embodiments, only the wax itself melts in the melt combination of the wax(es) and additives(s), and in other cases both the wax(es) and the additives(s) melt.

The pharmaceutical compositions of the present invention may be presented as a multi-layer tablet comprising a layer providing for the delayed release of one or more compounds of the invention, and a further layer providing for the immediate release of a medication for treatment of a disease or disorder contemplated in the invention. Using a wax/pH-sensitive polymer mix, a gastric insoluble composition may be obtained in which the active ingredient is entrapped, ensuring its delayed release.

### Parenteral Administration

As used herein, "parenteral administration" of a pharmaceutical composition includes any route of administration characterized by physical breaching of a tissue of a subject and administration of the pharmaceutical composition through the breach in the tissue. Parenteral administration thus includes, but is not limited to, administration of a pharmaceutical composition by injection of the composition, by application of the composition through a surgical incision, by application of the composition through a tissue-penetrating non-surgical wound, and the like. In particular, parenteral administration is contemplated to include, but is not limited to, subcutaneous, intravenous, intraperitoneal, intramuscular, intrastemal injection, and kidney dialytic infusion techniques.

Formulations of a pharmaceutical composition suitable for parenteral administration comprise the active ingredient combined with a pharmaceutically acceptable carrier, such as sterile water or sterile isotonic saline. Such formulations may be prepared, packaged, or sold in a form suitable for bolus administration or for continuous administration. Injectable formulations may be prepared, packaged, or sold in unit dosage form, such as in ampules or in multidose containers containing a preservative. Formulations for parenteral administration include, but are not limited to, suspensions, solutions, emulsions in oily or aqueous vehicles, pastes, and implantable sustained-release or biodegradable formulations. Such formulations may further comprise one or more additional ingredients including, but not limited to, suspending, stabilizing, or dispersing agents. In one embodiment of a formulation for parenteral administration, the active ingredient is provided in dry (*i.e.,* powder or granular) form for reconstitution with a suitable vehicle (*e.g.,* sterile pyrogen-free water) prior to parenteral administration of the reconstituted composition.

The pharmaceutical compositions may be prepared, packaged, or sold in the form of a sterile injectable aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally-acceptable diluent or solvent, such as water or 1,3-butanediol, for example. Other acceptable diluents and solvents include, but are not limited to, Ringer's solution, isotonic sodium chloride solution, and fixed oils such as synthetic mono- or di-glycerides. Other parentally-administrable formulations which are useful include those which comprise the active ingredient in microcrystalline form, in a liposomal preparation, or as a component of a biodegradable polymer system. Compositions for sustained release or implantation may comprise pharmaceutically acceptable polymeric or hydrophobic materials such as an emulsion, an ion exchange resin, a sparingly soluble polymer, or a sparingly soluble salt.

### Controlled Release Formulations and Drug Delivery Systems

In certain embodiments, the formulations for use according to the present invention may be, but are not limited to, short-term, rapid-offset, as well as controlled, for example, sustained release, delayed release and pulsatile release formulations.

The term sustained release is used in its conventional sense to refer to a drug formulation that provides for gradual release of a drug over an extended period of time, and that may, although not necessarily, result in substantially constant blood levels of a drug over an extended time period. The period of time may be as long as a month or more and should be a release which is longer that the same amount of agent administered in bolus form.

For sustained release, the compounds may be formulated with a suitable polymer or hydrophobic material that provides sustained release properties to the compounds. As such, the compounds useful within the methods of the invention may be administered in the form of microparticles, for example by injection, or in the form of wafers or discs by implantation.

In one embodiment of the invention, the compounds of the invention are administered to a patient, alone or in combination with another pharmaceutical agent, using a sustained release formulation.

The term delayed release is used herein in its conventional sense to refer to a drug formulation that provides for an initial release of the drug after some delay following drug administration and that may, although not necessarily, includes a delay of from about 10 minutes up to about 12 hours.

The term pulsatile release is used herein in its conventional sense to refer to a drug formulation that provides release of the drug in such a way as to produce pulsed plasma profiles of the drug after drug administration.

The term immediate release is used in its conventional sense to refer to a drug formulation that provides for release of the drug immediately after drug administration.

As used herein, short-term refers to any period of time up to and including about 8 hours, about 7 hours, about 6 hours, about 5 hours, about 4 hours, about 3 hours, about 2 hours, about 1 hour, about 40 minutes, about 20 minutes, about 10 minutes, or about 1 minute and any or all whole or partial increments thereof after drug administration after drug administration.

As used herein, rapid-offset refers to any period of time up to and including about 8 hours, about 7 hours, about 6 hours, about 5 hours, about 4 hours, about 3 hours, about 2 hours, about 1 hour, about 40 minutes, about 20 minutes, about 10 minutes, or about 1 minute and any and all whole or partial increments thereof after drug administration.

### Dosing

A suitable dose of a compound of the present invention may be in the range of from about 0.01 mg to about 5,000 mg per day, such as from about 0.1 mg to about 1,000 mg, for example, from about 1 mg to about 500 mg, such as about 5 mg to about 250 mg per day. The dose may be administered in a single dosage or in multiple dosages, for example from 1 to 5 or more times per day. When multiple dosages are used, the amount of each dosage may be the same or different. For example, a dose of 1 mg per day may be administered as two 0.5 mg doses, with about a 12-hour interval between doses.

It is understood that the amount of compound dosed per day may be administered, in non-limiting examples, every day, every other day, every 2 days, every 3 days, every 4 days, or every 5 days. For example, with every other day administration, a 5 mg per day dose may be initiated on Monday with a first subsequent 5 mg per day dose administered on Wednesday, a second subsequent 5 mg per day dose administered on Friday, and so on.

In the case wherein the patient's status does improve, upon the doctor's discretion the administration of the inhibitor of the invention is optionally given continuously; alternatively, the dose of drug being administered is temporarily reduced or temporarily suspended for a certain length of time (*i.e.,* a "drug holiday"). The length of the drug holiday optionally varies between 2 days and 1 year, including by way of example only, 2 days, 3 days, 4 days, 5 days, 6 days, 7 days, 10 days, 12 days, 15 days, 20 days, 28 days, 35 days, 50 days, 70 days, 100 days, 120 days, 150 days, 180 days, 200 days, 250 days, 280 days, 300 days, 320 days, 350 days, or 365 days. The dose reduction during a drug holiday includes from 10%-100%, including, by way of example only, 10%, 15%, 20%, 25%, 30%, 35%, 40%, 45%, 50%, 55%, 60%, 65%, 70%, 75%, 80%, 85%, 90%, 95%, or 100%.

Once improvement of the patient's conditions has occurred, a maintenance dose is administered if necessary. Subsequently, the dosage or the frequency of administration, or both, is reduced, as a function of the disease or disorder, to a level at which the improved disease is retained. In certain embodiments, patients require intermittent treatment on a long-term basis upon any recurrence of symptoms and/or infection.

The compounds for use in the method of the invention may be formulated in unit dosage form. The term "unit dosage form" refers to physically discrete units suitable as unitary dosage for patients undergoing treatment, with each unit containing a predetermined quantity of active material calculated to produce the desired therapeutic effect, optionally in association with a suitable pharmaceutical carrier. The unit dosage form may be for a single daily dose or one of multiple daily doses (*e.g.,* about 1 to 5 or more times per day). When multiple daily doses are used, the unit dosage form may be the same or different for each dose.

Toxicity and therapeutic efficacy of such therapeutic regimens are optionally determined in cell cultures or experimental animals, including, but not limited to, the determination of the LD₅₀ (the dose lethal to 50% of the population) and the ED₅₀ (the dose therapeutically effective in 50% of the population). The dose ratio between the toxic and therapeutic effects is the therapeutic index, which is expressed as the ratio between LD₅₀ and ED₅₀. The data obtained from cell culture assays and animal studies are optionally used in formulating a range of dosage for use in human. The dosage of such compounds lies preferably within a range of circulating concentrations that include the ED₅₀ with minimal toxicity. The dosage optionally varies within this range depending upon the dosage form employed and the route of administration utilized.

Those skilled in the art will recognize, or be able to ascertain using no more than routine experimentation, numerous equivalents to the specific procedures, embodiments, claims, and examples described herein. Such equivalents were considered to be within the scope of this invention and covered by the claims appended hereto. For example, it should be understood, that modifications in reaction conditions, including but not limited to reaction times, reaction size/volume, and experimental reagents, such as solvents, catalysts, pressures, atmospheric conditions, *e.g.,* nitrogen atmosphere, and reducing/oxidizing agents, with art-recognized alternatives and using no more than routine experimentation, are within the scope of the present application.

It is to be understood that, wherever values and ranges are provided herein, the description in range format is merely for convenience and brevity and should not be construed as an inflexible limitation on the scope of the invention. Accordingly, all values and ranges encompassed by these values and ranges are meant to be encompassed within the scope of the present invention. Moreover, all values that fall within these ranges, as well as the upper or lower limits of a range of values, are also contemplated by the present application. The description of a range should be considered to have specifically disclosed all the possible sub-ranges as well as individual numerical values within that range and, when appropriate, partial integers of the numerical values within ranges. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

The following examples further illustrate aspects of the present invention.

### EXAMPLES

The invention is now described with reference to the following Examples. These Examples are provided for the purpose of illustration only and the invention should in no way be construed as being limited to these Examples, but rather should be construed to encompass any and all variations which become evident as a result of the teaching provided herein.

### Materials and Methods

### Animals

Compulsive-like BIG male mice, *Mus musculus,* were raised on wood shavings in polypropylene cages (27x17x12 cm) under controlled temperature (22±1°C) and light (12:12 light-dark cycle) with free access to food (Purina Mills, Lab Diet Mouse Diet #5015, St. Louis, MO) and water. Animals were approximately 12-16 weeks of age at the start of each experiment.

### Drug Administration

Deformylflustrabromine hydrochloride (dFBr; Abeam Biochemicals) was dissolved in physiological saline (pH=6.7) to yield final doses of 2.0 mg/kg (0.27 mg/mL), 4 mg/kg (0.53 mg/mL) and 6 mg/kg (0.80 mg/mL). Saline was used as a vehicle control (0 mg/kg). A mouse of 40 g received an injection volume of 0.3 mL. Injection volumes were proportionally adjusted according to the body weight of individual animals.

***Acute Study*:** Male BIG mice were divided into four treatment groups comprising vehicle (sterile saline), 2 mg/kg, 4 mg/kg and 6 mg/kg. Animals in each group (n=12 per group) were tested for nesting on day 1, MB on day 3 and open field (OF) on day 5. On the first day of testing animals randomly received dFBr or vehicle subcutaneously and in subsequent tests received the same dose. Days 2 and 4 were employed to avoid any residual effects of dFBr from previous administration. For nesting, data were collected after 1, 2, 3, 4, 5 and 24 h due to the progressive nature of the NB (The BIG mice typically get excited and indulge in excessive and repetitive NB when introduced to cotton for the first 3-4 h in the light cycle. This excessive and repetitive nesting activity resumes again in the dark cycle). MB and OF behavior was performed 2 h after dFBr administration (FIG. 1).

***Chronic Study*;** Since the foundation of the animal model was established through effective reversal of compulsive-like NB and MB behaviors by chronic fluoxetine treatment, a chronic regimen was also conducted to establish the sustained and long term effects of dFBr on NB and MB. Animals belonging to 0, 2, 4 and 6 mg/kg dose group (n=12 per group) received single subcutaneous injection of dFBr or saline daily for 32 days. NB, MB and OF behaviors were assessed in the final week (weeks 5) after dFBr administration (NB after 1, 2, 3, 4, 5 and 24 h, and MB after 2 h of drug injection). NB was performed on day 30, MB on day 31 and OF on day 32 (FIG. 1).

The dosages and route of administration was determined based on the fact that dFBr penetrates the blood-brain barrier and reaches the brain amounting to around 36% in the cerebrospinal fluid after 90 min of subcutaneous administration.

### Assessment of Compulsive-like Behavior

### Nest Building Behavior

Nest-building behavior was performed to assess compulsive-like behavior in the mice. For both acute and chronic studies, immediately following subcutaneous injection of dFBr, compulsive-like male mice were singly housed and provided with a pre-weighed roll of cotton (Mountain Mist cotton batting, Troy, Inc., Chicago, Illinois) in the cage-top food hopper. The cotton roll was weighed after 1, 2, 3, 4, 5 and 24 hours. Nesting behavior was quantified by the grams of cotton used during each testing period.

### Marble Burying Behavior

The marble-burying test is an effective test for determining compulsive-like behavior in mice. Mice generally do not interact with the marbles, and thus the MB teste measures only digging behavior. Two hours after dFBr administration, compulsive-like male mice were individually introduced to a polypropylene cage (37x21x14 cm) containing 20 glass marbles (10 mm in diameter) evenly spaced on 5 cm deep wood chip bedding without access to food or water for 20 min. Testing was carried out in the testing room separate from the housing room. The total number of marbles buried at least 2/3 in the 20-min period was quantified as compulsive-like digging behavior. After the 20-min test, the animals were returned to their home cages.

### Assessment of Locomotory and Anxiety-like Behavior

### Open Field Test

Anxiety-like behaviors were determined through the open field test. Compulsive-like male mice were individually introduced into an open field (40x40x35 cm) with a central zone (20x20 cm). The apparatus was placed underneath an overhead light illuminating the entire open field. The animals were placed in the center of the open field, and their behavior was video taped for 3 min and analyzed with the aid of ANYMAZE^{™} video tracking software (Stoelting Co., IL). The time spent in the center (anxiety-like measure) and total distance travelled (locomotion) in the entire open field were measured. The open field was cleaned before each test. For the BIG mice in OF, a 3 min duration provides consistent outcomes for assessment of locomotory and anxiety-like behaviors and therefore considered for the current experiment.

### Statistical Analysis

Statistical analysis was performed in Graphpad Prism (GraphPad Software, Inc) and Statistical Analysis System Software (Version 9.4, Cary, NC). Nest-building (grams of cotton), marble burying (number of marbles at least 2/3 buried) and open field measures (time in center and total distance travelled) were expressed as the mean ± standard error of the mean (SEM). The nest building data were shown as grams of cotton used, whereas the statistical analysis was conducted on the square-root transformed nesting scores in order to normalize the data (Bult & Lynch, 2000, Behavior Genetics 30:193-206). Nesting scores at different time points, marble burying and open field results were analyzed by one-way analysis of variance (ANOVA), whereas overall drug and drug by time interaction effect between 0-5 hours was done by two-way analysis of variance (ANOVA). Pairwise comparisons for significant differences were tested by the post-hoc Bonferroni multiple comparison test. A probability level of *p*<0.05 was used as an index of statistical significance in all cases.

### Example 1: Compulsive-like nesting behavior (NB) during the first 5 hours (FIG. 2A)

Two-way ANOVA for time versus nesting score for different concentrations (FIGs. 2A-2B) revealed that there was a significant drug (*F*_{(3,220)}=38.60,*p*<0.0001) effect during the first 5 hours, a significant time (*F*_{(4,220)}=44.71, *p*<0.0001) and drug by time interaction effect (*F*_{(12,220)}=7.08, *p*<0.0001) in the compulsive-like nesting behavior.

Following 1 hour of dFBr administration, there was no significant attenuation of nesting (*F*_{(3,44)}=1.276,*p*>0.29) by the 2 mg/kg (*t*₂₂=0.01422, *p*>0.98), 4 mg/kg (*t*₂₂=1.633, *p>0.11)* and 6 mg/kg (*t*₂₂=0.9985, *p*>0.32) doses.

Between 1 and 2 hours, dFBr administration resulted in dose-dependent and significant reductions in compulsive-like nesting (*F*_{(3,44)}=26.42, *p*<0.0001). The Bonferroni's post-hoc test revealed that the 4 mg/kg (*t*₂₂=6.210, *p*<0.001) and 6 mg/kg (*t*₂₂=6.638, *p*<0.001) doses of dFBr significantly attenuated nesting as compared to the control (saline). The 2 mg/kg dose showed no significant effect when compared to the control (*t*₂₂=0.2771, *p*>0.78). The nesting scores of the mice exposed to 4 mg/kg and 6 mg/kg doses of dFBr were also significantly less than the 2 mg/kg dose (*t*₂₂=5.933, *p*<0.001; *t*₂₂=6.360, *p*<0.001, respectively).

Between 2 and 3 hours after dFBr administration, nesting scores was significantly reduced (*F*_{(3,44)}=7.906,*p*<0.0005) in the 6 mg/kg (*t*₂₂=3.727, *p*<0.01) group when compared to the control group. No significant difference was observed for the 4 mg/kg (*t*₂₂=2.686, *p*>0.05) and 2 mg/kg group (*t*₂₂=0.3012,*p*>0.76). There was also significant attenuation of nesting in 6 mg/kg (*t*₂₂=4.029, *p*<0.01) group when compared to the 2 mg/kg.

Between 3 and 4 hours after dFBr administration, the nesting score was significantly reduced (*F*_{(3,44)}=8.094, *p*<0.0005) in the 4 mg/kg (*t*₂₂=3.305, *p*>0.05) and 6 mg/kg (*t*₂₂=4.585, *p*<0.001) group compared to the control group. Nesting in the 2 mg/kg group was not significantly different (*t*₂₂=1.507, *p*>0.14). The 6 mg/kg group reduced nesting significantly (*t*₂₂=3.078, *p*<0.05) when compared to the 2 mg/kg group.

Between 4 and 5 hours after dFBr administration, nesting scores were not significantly different (*F*_{(3,44)}=2.375, *p*>0.05) for the 2 mg/kg, 4 mg/kg and 6 mg/kg groups (*t*₂₂=1.592, *p*>0.12, *t*₂₂=1.972, *p*>0.06 and *t*₂₂=2.541, *p*>0.05, respectively).

Overall, between 0 and 5 hours, the nesting score was significantly reduced (*F*_{(3,44)}=22.94, *p*>0.0001), with the 4 mg/kg (*t*₂₂=5.431, *p*<0.001) and 6 mg/kg (*t*₂₂=6.277, *p*<0.001) groups than the control group, while the 2 mg/kg (*t*₂₂=0.03715, *p*>0.96) group did not attenuate nesting. The 4 mg/kg (*t*₂₂=5.393, *p*<0.001) and 6 mg/kg (*t*₂₂=6.239, *p*<0.001) groups attenuated nesting significantly when compared to the 2 mg/kg group (FIGs. 2A-2B).

Between 5-24 hours after dFBr administration, the nesting scores declined (*F*_{(3,44)}=7.645, *p*<0.0006), but all three doses had a similar effect. The 2 mg/kg (*t*₂₂=4.213, *p*<0.01), 4 mg/kg (*t*₂₂=3.656, *p*<0.01) and 6 mg/kg (*t*₂₂=3.772, *p*<0.01) doses supressed the nesting score significantly when compared to the control group.

### Example 2: Compulsive-like nesting behavior (NB) between 0-24 hours in acute administration (FIG. 5A)

24 hours after dFBr administration, overall nesting scores (time 0 through 24 hours) were dose-dependently and significantly reduced (*F*_{(3,44)}=7.645, *p*<0.0006) with the 2 mg/kg (*t*₂₂=6.213, *p*<0.001), 4 mg/kg (*t*₂₂=9.774, *p*<0.001), and 6 mg/kg (*t*₂₂=10.50, *p*<0.001) groups significantly below the control group. There was also significant reduction in nesting in the 6 mg/kg group when compared to 2 mg/kg group (*t*₂₂=2.994, *p*<0.05) (FIG. 3).

Overall, the dose dependent effect of dFBr by fractional nesting score (computed by dividing each individual nesting score of the three dfbr dose groups by the average scores of the vehicle group at each time point) showed that the 4 mg/kg and 6 mg/kg doses suppressed nesting the most 1-2 hours after administration (FIG. 4). This supression of compulsive-like nesting gradually decreased during the next three hours. The 2 mg/kg dose had fractional scores close to 1 during the first 5 hours, but statistical significance was reached after the 5 hour time point during the remainer of the 24 hour testing period (FIG. 3).

### Example 3: Compulsive-like nesting behavior (NB) during 0-5 hours in chronic administration (FIG. 2B)

Significant drug (*F*_{(3,220)}=4.87, *p*<0.01) time (*F*_{(4,220)}=177.12, *p*<0.0001) and drug by time interaction effect (*F*_{(12,220)}=2.29, *p*<0.01) was observed in the first 5 h of the chronic administration. Between 0 h and 1 h there was an overall suppression of NB (*F*_{(3,44)}=6.52, *p*<0.01) with 4 mg/kg and 6mg/kg being the most effective doses (*t*₂₂=6.097, *p*<0.001 and *t*₂₂=5.394, *p*<0.001 respectively). For 1-2 h the NB declined (*F*_{(3,44)}=4.86, *p*<0.01) significantly with 2 and 6 mg/kg showing the main attenuating effects (*t*₂₂=3.086, *p*<0.05 and *t*₂₂=3.210, *p*<0,01 respectively). No significant effect was observed for NB between 2-3 h (*F*_{(3,44)}=1.54, NS), 3-4 h (*F*_{(3,44)}=1.01, NS) and 4-5 h (*F*_{(3,44)}=6.52, NS).

### Example 4: Compulsive-like nesting behavior (NB) during 0-24 hours in chronic administration (FIG. 5B)

24 hours (time 0 through 24 h) after dFBr administration, overall nesting scores were dose-dependently and significantly reduced (*F*_{(3,44)}=8.85, *p*<0.0001) with the 2 mg/kg (*t*₂₂=4.574, *p*<0.05), 4 mg/kg (*t*₂₂=7.149, *p*<0.001) and 6 mg/kg (*t*₂₂=4.555, *p*<0.05) groups significantly below the control group.

### Example 5: Compulsive-like marble burying (MB) behavior Acute Administration (FIG. 6A):

Marble-burying behavior of the compulsive-like male mice were significantly reduced (*F*_{(3,44)}=64.62, *p*<0.0001) 2 hours after dFBr administration. The 2 mg/kg, 4 mg/kg and 6 mg/kg doses decreased marble-burying behavior dose-dependently compared to the control (*t*₂₂=3.428, *p*<0.05; *t*₂₂=12.85*, p*<0.001; *t*₂₂=7.667, *p*<0.001, respectively). The 4 mg/kg and 6 mg/kg doses also attenuated marble-burying behavior more than the 2 mg/kg dose (*t*₂₂=9.426, *p*<0.001, and *t*₂₂=5.332, *p*<0.001, respectively).

### Chronic Administration (FIG. 6B):

dFBr suppressed MB behavior significantly (*F*_{(3,44)}= 40.03, *p*<0.0001) in the fifth week of administration. The most effective doses were 4 mg/kg and 6 mg/kg, which showed the maximum suppression of MB when compared to control (*t*₂₂=8.643, *p*<0.001; *t*₂₂=8.554, *p*<0.001, respectively). The 4 and 6 mg/kg doses were also significantly lower than the 2 mg/kg dose (*t*₂₂=7.039, *p*<0.001; *t*₂₂=6.950, *p*<0.001, respectively).

### Example 4: Anxiety-like open field (OF) behavior (FIGs. 7A-7B and 8A-8B) Acute Administration

The total distance traveled, which is used to quantify locomotor activity, was not different among the treatment groups (*F*_{(3,44)}=1.213, NS; FIG. 7A). No significant differences were also observed among the treatment groups for the time spent in center of the OF (*F*_{(3,44)}=0.9849, NS; FIG. 8A).

### Chronic Administration

For the chronic regimen the total distance (*F*_{(3,44)}=0.30, NS) and time in center (*F*_{(3,44)}=0.18, NS) did not differ among treatment groups (FIGs. 7B and 8B).

### Example 5:

As demonstrated herein, administration of an illustrative non-limiting α4β2 PAM, dFBr, produced a reduction incompulsive-like NB and MB, but did not alter anxiety-like and locomotor activity in the OF for the acute study. A similar response to chronic dFBr was observed where the treatment groups showed rapid suppression of NB (1 h and 2 h) and MB (2 h) after dFBr administration. OF behaviors however remained unaffected by the chronic treatment. These results indicate an apparent selectivity of dFBr for compulsive-like behaviors, indicating that potentiation of α4β2 nAChRs is an alternative approach for suppressing compulsive-like phenotype, thereby posing significant translational potential.

In the acute administration, 4 mg/kg and 6 mg/kg dFBr doses had the largest attenuating effects on NB 2 h after injection, while for the chronic administration the suppression effects on NB was visible after the first hour and endured in the second hour with 6 mg/kg showing a more consistent effect. An earlier effectof dFBr (1 h after administration) on NB was observed for the chronic study, indicating in a non-limiting embodiments sensitization to dFBr due to repeated treatment. The attenuating effects gradually decreased during the next 4 h for both the treatment, showing that dFBr had a rapid effect. This result is consistent with the finding that peak levels of dFBr in the cerebrospinal fluid occur 90 min after administration in rats. The 2 mg/kg dFBr dose had no immediate attenuating effect on NB. A long term effect of this dose was however seen in both acute (after 24 h) and chronic (week 5) administration, indicating that this dose was effective over a longer time period.

The effects of dFBr, 2 h after injection, on MB behavior were generally similar to the effects on NB. However, at the 2 h time point in the acute treatment 2 mg/kg moderately and significantly reduced MB behavior. This effect was not significant in the chronic regimen. No significant effect was observed on NB at the same dose and time point in the acute study, but had an effect in the chronic study. Without wishing to be limited by any theory, these different effects of dFBr treatment may indicate subtle differences in the brain mechanisms that control NB and MB behavior. Clinical studies have shown that some OCD patients with specific types of symptoms do not respond to first line therapies in a similar way. The doses that act to attenuate obsessions and compulsions in general OCD patients typically fail to produce results in treatment resistant ones. Moreover, recommended doses for first line treatments might vary depending on the severity of the disorder, co-morbid symptoms like anxiety and potential side effects. Though a common agreement on OCD subtypes is lacking, therapeutic response and results for each OCD subtype are different. For example, fluoxetine, a common OCD drug, has greater efficacy in washers and obsessive thoughts when compared to checkers. Without wishing to be limited by any theory, the variation in dose response to dFBr of compulsive-like MB and NB behavior adds additional heterogeneity to the BIG mouse for assessing drug effects on various compulsive-like phenotypes.

Acute and chronic dFBr regimen failed to modulate anxiety-like (time spent in center) and locomotor (total distance traveled) behaviors in the OF test. SSRIs like fluoxetine failed to reduce overall wheel-running locomotion in the compulsive-like BIG mice but significantly attenuated NB and MB behavior (Greene-Schloesser, et al., 2011, Behav. Brain Res. 221:55-62). Without wishing to be limited by any theory, separate brain regions and signaling pathways influencing compulsive-like and anxiety-like symptoms are a likely explanation for the observed lack of a dFBr effect in the OF test. Anxiety is attributed primarily to the amygdala and ventral hippocampus, whereas compulsions and obsessions have been linked to dorsolateral prefrontal cortex, anterior cingulate cortex, orbitofrontal cortex and dysregulation of the corticostriatal-thalamo-cortical circuitry (CSTC). These regions receive projections from the amygdala and hippocampus, explaining the co-existence of anxiety along with OCD, which appears to be specific to anxiety related to compulsive-like behaviors rather than more generalized anxiety.

Removal or inhibition by antagonists of α4β2 nAChRs abolishes the anxiolytic effects of nicotine, while stimulating these nAChRs receptors with an agonist decreases anxiety-like behavior. In contrast, anxiogenic effects of nicotine withdrawal are enhanced by stimulation of α7 nAChRs and decreased by inhibition of these nAChRs receptors. Allosteric modulation of α4β2 nAChRs by dFBr did not affect anxiety-like behavior in the OF test in the BIG mice. Without wishing to be limited by any theory, these nAChRs receptors may not be involved in the control of anxiety in nicotine-naive mice.

As described herein, both acute and chronic dFBr was effective in reversing compulsive-like NB and MB,without exerting any influence on anxiety-like and locomotory behaviors. This indicates the therapeutic potential of modulation of α4β2 nAChRs by dFBr, or any other positive allosteric modulator of this receptor, for compulsive phenotypes. Due to the rapid rate of onset (a few hours) of the attenuating effects of dFBr on compulsive-like behaviors, this class of specific nicotinic subtype modulators can also provide more immediate suppression effects, thereby providing a bridging option to other first line therapies (*e.g*., SSRIs) that display longer time courses for onset of effectiveness. dFBr maintained its attenuating effects on NB and MB during chronic treatment, and can thus also represent a novel first line treatment.

## Claims

1. Desformylflustrabromine, a salt or solvate thereof for use in a method of treating and/or preventing obsessive-compulsive and related disorders (OCRD) in a subject in need thereof.

2. Desformylflustrabromine, a salt or solvate thereof for use according to claim 1, wherein the desformylflustrabromine is the only therapeutically effective compound for administration in a therapeutically sufficient amount to treat and/or prevent obsessive-compulsive and related disorders (OCRD).

3. Desformylflustrabromine, a salt or solvate thereof for use according to claim 1, wherein (i) the subject had not previously received any pharmacological treatment for the obsessive-compulsive and related disorders (OCRD), or (ii) the subject had previously received another pharmacological treatment for the obsessive-compulsive and related disorders (OCRD).

4. Desformylflustrabromine, a salt or solvate thereof for use according to claim 1, wherein the desformylflustrabromine is for chronic and/or acute administration to the subject.

5. Desformylflustrabromine, a salt or solvate thereof for use according to claim 1, wherein one or more additional agents known to treat obsessive-compulsive and related disorders (OCRD) are for further administration to the subject.

6. Desformylflustrabromine, a salt or solvate thereof for use according to claim 5, wherein the one or more additional agents are selected from the group consisting of clomipramine, fluvoxamine, fluoxetine, paroxetine, buspirone, sertraline, citalopram, escitalopram venlafaxine, dapoxetine, hydrocodone, D-cycloserine, buspirone, clonazepam, trazodone, tranylcypromine, venlafaxine, olanzapine, L-tryptophan, pindolol, gabapentin, lorazepam, bupropion, amantadine, methylphenidate, dexedrine, yohimbine, sildenafil, mirtazapine, nefazodone, valproate, lithium, risperidone, phenelzine, haloperidol, pimozide, aripiprazole, tramadol, N-acetylcysteine, topiramate, lamotrigine, and inositol.

7. Desformylflustrabromine, a salt or solvate thereof for use according to claim 1, wherein the desformylflustrabromine is for administration to the subject by at least one route selected from the group consisting of oral, nasal, inhalational, topical, buccal, rectal, pleural, peritoneal, vaginal, intramuscular, subcutaneous, transdermal, epidural, intratracheal, otic, intraocular, intrathecal, and intravenous routes.

8. Desformylflustrabromine, a salt or solvate thereof for use according to claim 1, wherein the desformylflustrabromine is for administration as part of a pharmaceutical composition.

9. Desformylflustrabromine, a salt or solvate thereof for use according to claim 1, wherein the therapeutically effective amount of desformylflustrabromine ranges from 0.001 mg/day to 1,000 mg/day.

10. Desformylflustrabromine, a salt or solvate thereof for use according to claim 1, wherein the subject is a mammal or bird.

11. Desformylflustrabromine, a salt or solvate thereof for use according to claim 1, wherein the subject is a pet, livestock or human.

12. Desformylflustrabromine, a salt or solvate thereof for use according to claim 1, wherein the subject further has an autism spectrum disorder (ASD).

13. Desformylflustrabromine, a salt or solvate thereof for use according to claim 1, wherein the obsessive-compulsive and related disorders (OCRD) is obsessive-compulsive disorder (OCD).

## Patentansprüche

1. Desformylflustrabromin, ein Salz oder Solvat davon zur Verwendung in einem Verfahren zur Behandlung und/oder Vorbeugung von Zwangsstörung bzw. einer verwandten Erkrankung (OCRD) in einem Subjekt, das dessen bedarf.

2. Desformylflustrabromin, ein Salz oder Solvat davon zur Verwendung gemäß Anspruch 1, wobei Desformylflustrabromin die einzige therapeutisch wirksame Verbindung zur Verabreichung in einer therapeutisch ausreichenden Menge ist, um die Zwangsstörung bzw. verwandte Erkrankung (OCRD) zu behandeln und/oder zu verhindern.

3. Desformylflustrabromin, ein Salz oder Solvat davon zur Verwendung gemäß Anspruch 1, wobei (i) das Subjekt zuvor keine pharmakologische Behandlung für die Zwangsstörung bzw. verwandte Erkrankung (OCRD) erhalten hat oder (ii) das Subjekt zuvor eine andere pharmakologische Behandlung für die Zwangsstörung bzw. verwandte Erkrankung (OCRD) erhalten hat.

4. Desformylflustrabromin, ein Salz oder Solvat davon zur Verwendung gemäß Anspruch 1, wobei Desformylflustrabromin zur chronischen und/oder akuten Verabreichung an den Patienten bestimmt ist.

5. Desformylflustrabromin, ein Salz oder Solvat davon zur Verwendung gemäß Anspruch 1, wobei ein oder mehrere zusätzliche Mittel, die zur Behandlung von Zwangsstörung bzw. verwandte Erkrankung (OCRD) bekannt sind, zur weiteren Verabreichung an den Patienten vorgesehen sind.

6. Desformylflustrabromin, ein Salz oder Solvat davon zur Verwendung gemäß Anspruch 5, wobei das eine oder mehrere zusätzliche Mittel ausgewählt sind aus der Gruppe bestehend aus Clomipramin, Fluvoxamin, Fluoxetin, Paroxetin, Buspiron, Sertralin, Citalopram, Escitalopram, Venlafaxin, Dapoxetin, Hydrocodon, D-Cycloserin, Buspiron, Clonazepam, Trazodon, Tranylcypromin, Venlafaxin, Olanzapin, L-Tryptophan, Pindolol, Gabapentin, Lorazepam, Bupropion, Amantadin, Methylphenidat, Dexedrin, Yohimbin, Sildenafil, Mirtazapin, Nefazodon, Valproat, Lithium, Risperidon, Phenelzin, Haloperidol, Pimozid, Aripiprazol, Tramadol, N-Acetylcystein, Topiramat, Lamotrigin und Inositol.

7. Desformylflustrabromin, ein Salz oder Solvat davon zur Verwendung gemäß Anspruch 1, wobei Desformylflustrabromin zur Verabreichung an das Subjekt über mindestens einen Weg ausgewählt aus der Gruppe bestehend aus oralen, nasalen, inhalativen, topischen, bukkalen, rektalen, pleuralen, peritonealen, vaginalen, intramuskulären, subkutanen, transdermalen, epiduralen, intratrachealen, otischen, intraokularen, intrathekalen und intravenösen Wegen bestimmt ist.

8. Desformylflustrabromin, ein Salz oder Solvat davon zur Verwendung gemäß Anspruch 1, wobei Desformylflustrabromin zur Verabreichung als Teil einer pharmazeutischen Zusammensetzung bestimmt ist.

9. Desformylflustrabromin, ein Salz oder Solvat davon zur Verwendung gemäß Anspruch 1, wobei die therapeutisch wirksame Menge von Desformylflustrabromin im Bereich von 0,001 mg/Tag bis 1.000 mg/Tag liegt.

10. Desformylflustrabromin, ein Salz oder Solvat davon zur Verwendung gemäß Anspruch 1, wobei das Subjekt ein Säugetier oder ein Vogel ist.

11. Desformylflustrabromin, ein Salz oder Solvat davon zur Verwendung gemäß Anspruch 1, wobei das Subjekt ein Haustier, ein Nutztier oder ein Mensch ist.

12. Desformylflustrabromin, ein Salz oder Solvat davon zur Verwendung gemäß Anspruch 1, wobei das Subjekt außerdem eine Autismus-Spektrum-Störung (ASD) hat.

13. Desformylflustrabromin, ein Salz oder Solvat davon zur Verwendung gemäß Anspruch 1, wobei die Zwangsstörung bzw. verwandte Erkrankung (OCRD) eine Zwangsstörung (OCD) ist.

## Revendications

1. Desformylflustrabrome, son sel ou solvate pour l'utilisation dans un procédé de traitement et/ou de prévention des troubles obsessivo-compulsifs et apparentés (OCRD) chez un sujet en ayant besoin.

2. Desformylflustrabrome, son sel ou solvate pour l'utilisation selon la revendication 1, le desformylflustrabrome étant l'unique composé thérapeutiquement efficace d'administration en une quantité thérapeutiquement suffisante pour traiter et/ou prévenir des troubles obsessivo-compulsifs et apparentés (OCRD).

3. Desformylflustrabrome, son sel ou solvate pour l'utilisation selon la revendication 1, (i) le sujet n'ayant précédemment pas reçu de quelconque traitement pharmacologique pour des troubles obsessivo-compulsifs et apparentés (OCRD), ou (ii) le sujet ayant précédemment reçu un autre traitement pharmacologique pour les troubles obsessivo-compulsifs et apparentés (OCRD).

4. Desformylflustrabrome, son sel ou solvate pour l'utilisation selon la revendication 1, le desformylflustrabrome étant une administration chronique et/ou aiguë au sujet.

5. Desformylflustrabrome, son sel ou solvate pour l'utilisation selon la revendication 1, un ou plusieurs agents additionnels connus pour traiter des troubles obsessivo-compulsifs et apparentés (OCRD) servant à une administration supplémentaire au sujet.

6. Desformylflustrabrome, son sel ou solvate pour l'utilisation selon la revendication 5, lesdits agents additionnels étant sélectionnés dans le groupe constitué de clomipramine, fluvoxamine, fluoxétine, paroxétine, buspirone, sertraline, citalopram, escitalopram venlafaxine, dapoxétine, hydrocodone, D-cyclosérine, buspirone, clonazépam, trazodone, tranylcypromine, venlafaxine, olanzapine, L-tryptophane, pindolol, gabapentine, lorazépam, bupropion, amantadine, méthylphénidate, dexédrine, yohimbine, sildénafil, mirtazapine, néfazodone, valproate, lithium, rispéridone, phénelzine, halopéridol, pimozide, aripiprazole, tramadol, N-acétylcystéine, topiramate, lamotrigine, et d'inositol.

7. Desformylflustrabrome, son sel ou solvate pour l'utilisation selon la revendication 1, le desformylflustrabrome servant à l'administration au sujet par au moins une voie sélectionnée dans le groupe constitué de l'administration par voie orale, nasale, par inhalation, topique, buccale, rectale, pleurale, péritonéale, vaginale, intramusculaire, sous-cutanée, transdermique, épidurale, intratrachéenne, otique, intraoculaire, intrathécale, et intraveineuse.

8. Desformylflustrabrome, son sel ou solvate pour l'utilisation selon la revendication 1, le desformylflustrabrome étant destiné à l'administration comme partie d'une composition pharmaceutique.

9. Desformylflustrabrome, son sel ou solvate pour l'utilisation selon la revendication 1, la quantité thérapeutiquement efficace de desformylflustrabrome s'étendant de 0,001 mg/jour à 1 000 mg/jour.

10. Desformylflustrabrome, son sel ou solvate pour l'utilisation selon la revendication 1, le sujet étant un mammifère ou un oiseau.

11. Desformylflustrabrome, son sel ou solvate pour l'utilisation selon la revendication 1, le sujet étant un animal domestique, un animal d'élevage ou un être humain.

12. Desformylflustrabrome, son sel ou solvate pour l'utilisation selon la revendication 1, le sujet présentant en outre un trouble du spectre autistique (ASD).

13. Desformylflustrabrome, son sel ou solvate pour l'utilisation selon la revendication 1, les troubles obsessivo-compulsifs et apparentés (OCRD) étant un trouble obsessivo-compulsif (OCD).
